# EUROPÄISCHE PATENTSCHRIFT

(11) **EP 0 749 954 B1**
(45) Veröffentlichungstag und Bekanntmachung des Hinweises auf die Patenterteilung: **09.06.1999**
(21) Anmeldenummer: 96109251.7
(22) Anmeldetag: 10.06.1996
(51) Int. Cl.: C07C 67/307, C07C 69/63

(54) **Verfahren zur Herstellung von 2-Fluor-isobuttersäure-alkylestern**
Process for producing 2-fluoroisobutyric acid esters
Procédé pour la préparation d'esters de l'acide 2-fluoroisobutyrique

(30) Priorität: 22.06.1995 DE 19522703
(43) Veröffentlichungstag der Anmeldung: 27.12.1996
(73) Patentinhaber: BAYER AG, 51368 Leverkusen (DE)
(72) Erfinder: Bertsch, Achim, Dr., 51061 Köln (DE)

(56) Entgegenhaltungen:
- EP-A- 0 506 059
- EP-A- 0 645 365
- DE-A- 4 131 242

## Beschreibung

Die vorliegende Erfindung betrifft ein Verfahren zur Herstellung von 2-Fluor-isobuttersäurealkylestern, die wichtige Zwischenprodukte für die Herstellung von Fungiziden und Herbiziden sind (siehe z.B. EP-A1 0 381 330 und WP 90/09378).

In der Literatur sind eine Reihe Laborsynthesen für 2-Fluor-isobuttersäureester beschrieben, die aber alle mit erheblichen Nachteilen behaftet sind, wenn sie in den technischen Maßstab übertragen werden sollen.

2-Fluor-isobuttersäureester können z.B. aus 2-Brom-isobuttersäureestern durch Halogenaustausch mit Silberfluorid bei 140°C erhalten werden (siehe J. Org. Chem. 33 4279 (1968)). Nachteilig hierbei sind die geringe Selektivität von nur 20 % und der hohe Preis für Silberfluorid.

Die EP-A1 0 509 544 beschreibt eine analoge Umsetzung mit Kaliumfluorid in 2,3-Butandiol bei 120°C. Die maximale, nur gaschromatographisch bestimmte Ausbeute beträgt 45 %. Störend ist die überwiegende Bildung von Methacrylsäureestern durch eine Eliminierungsreaktion.

In der EP-A1 0 509 544 ist auch die Addition von Fluorwasserstoff an Methacrylsäuremethylester beschrieben. Die maximale Ausbeute beträgt nur 25 %. Nachteile dieses Verfahrens, welches zwar von preiswerten Edukten ausgeht, sind neben der geringen Ausbeute die Verwendung von aufwendig aufzuarbeitenden, giftigen und ätzenden Fluorwasserstoff-Pyridin-Gemischen und Reaktionstemperaturen, bei denen Autoklaven aus korrosionsbeständigen Werkstoffen benutzt werden müssen. Bei Umsetzung in wasserfreiem Fluorwasserstoff, der eine einfache Wiedergewinnung erlaubt, sinkt die Ausbeute auf nur noch 16 %.

Die EP-A1 0 506 059 beschreibt die Synthese von 2-Fluor-isobuttersäureestern ausgehend von 2-Hydroxy-isobuttersäureestern durch Umsetzung mit Fluor- oder Chlorsulfonsäure und Fluorwasserstoff oder Fluorwasserstoff/Pyridin-Mischunngen. Nachteilig hierbei ist die Aufarbeitung des große Mengen an Fluorwasserstoff und gegebenenfalls Pyridin enthaltenen Abwassers Daneben stört hier wieder die Bildung erheblicher Mengen Methacrylsäureester, was von der Anmelderin selbst in der späteren Patentanmeldung WO 94/24086 angegeben wird.

In der EP-A1 0 468 681 wird der direkte Austausch der Hydroxy-Gruppe gegen Fluor bei 2-Hydroxy-2,2-dialkylessigestern mit Schwefeltetrafluorid oder Diethylaminoschwefeltrifluorid als Reagenz beschrieben. Den hervorragenden Ausbeuten steht hier die Verwendung des giftigen und in technischen Mengen nicht verfügbaren Schwefeltetrafluorids entgegen.

In der WO 94/24086 wird die Herstellung von 2-Fluor-isobuttersäureestern aus 2-Hydroxy-isobuttersäureestern in einem Zweistufenprozeß durch Umsetzung mit Thionylchlorid, gefolgt von einer Umsetzung mit Fluorwasserstoff oder Fluorwasserstoff/Amin-Gemischen beschrieben. Von Nachteil ist wiederum die Verwendung von Fluorwasserstoff, insbesondere im Gemisch mit Aminen. Darüberhinaus erfordert der hier anfallende chlorwasserstoffhaltige Fluorwasserstoff sehr teure Spezialwerkstoffe, um die wäßrige Aufarbeitung durchführen zu können.

In Tetrahedron Asymmetry 5, 981 (1994) ist die Fluorieiung von in 2-Position wasserstoffsubstituierten, d.h. sekundären 2-Alkyl-2-sulfonyloxyessigsäureestern beschrieben. Der Erhalt der Enantiomerenreinheit bei Einsatz von chiralen Edukten wie z.B. (S)-Milchsäureester beweist hier den Verlauf der Reaktion unter Konfigurationsumkehr über einen S_{N}2-Mechanismus.

Es wurde nun ein Verfahren zur Herstellung von 2-Fluorisobuttersäurealkylestern der Formel (I) gefunden, 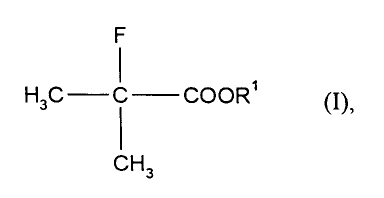 in der
- R¹: einen gegebenenfalls verzweigten und/oder gegebenenfalls fluorierten Alkylrest mit 1 bis 4 Kohlenstoffatomen bedeutet,
das dadurch gekennzeichnet ist, daß man einen Sulfonsäureester der Formel (II) 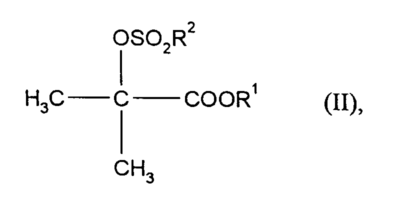 in der
- R¹: die bei Formel (I) angegebene Bedeutung und
- R²: unabhängig von R¹ den gleichen Bedeutungsumfang wie R¹ hat,
mit Kaliumfluorid in einem Lösungsmittel der Formel (III)

R³-CO-NHR⁴ (III),

in der
- R³ und R⁴: jeweils unabhängig voneinander Wasserstoff, Phenyl oder Alkyl mit 1 bis 4 Kohlenstoffatomen bedeutet,
umsetzt.

Beispiele für die Reste R¹ und R² sind Methyl, Trifluormethyl, Ethyl, n-Propyl, i-Propyl, n-Butyl, i-Butyl und tert.-Butyl. Bevorzugt sind Methyl und Ethyl. Besonders bevorzugt wird 2-Methansulfonyloxy-isobuttersäuremethylester eingesetzt und daraus 2-Fluor-isobuttersäuremethylester hergestellt.

Sulfonsäureester der Formel (II) sind leicht in an sich bekannter Weise herstellbar, z.B. aus den entsprechenden 2-Hydroxy-isobuttersäureestern durch Umsetzung mit Säurechloriden oder -anhydriden, die aus dem Rest R² und einer Chlorsulfon-oder einer Sulfonsäureanhydridgruppe bestehen. Diese Reaktion wird vorzugsweise in Gegenwart von Basen durchgeführt. Als Basen kommen z.B. organische Basen wie tert.-Amine oder anorganische Basen wie Natriumbicarbonat in Frage.

Das Kaliumfluorid kann in handelsüblicher Form eingesetzt werden. Vorzugsweise ist es feingepulvert.

Die Sulfonsäureester der Formel (II) werden vorzugsweise mit einem Überschuß von Kaliumfluorid versetzt. Geeignet sind z.B. Mengen von 1,05 bis 20 Mol, insbesondere 2 bis 5 Mol Kaliumfluorid pro Mol Sulfonsäureester der Formel (II).

Bevorzugte Lösungsmittel der Formel (III) sind solche, bei denen R³ und R⁴ jeweils unabhängig voneinander Wasserstoff oder Methyl bedeuten. Besonders bevorzugt ist Formamid. Es können auch Gemische von Lösungsmitteln der Formel (III) und weiteren Lösungsmitteln eingesetzt werden, wobei solche Gemische vorzugsweise mindestens 20 Gew.-% eines oder mehrerer der Lösungsmittel der Formel (III) enthalten. Es ist nicht zwingend erforderlich, daß sich alle Reaktionskomponenten vollständig in dem Lösungsmittel oder in dem Lösungsmittelgemisch lösen müssen. Die Lösungsmittel können z.B. in der 2-bis 5fachen Gewichtsmenge, bezogen auf Sulfonsäureester der Formel (II) eingesetzt werden.

Die Reaktionstemperatur kann z.B. im Bereich 40 bis 140°C liegen. Bevorzugt beträgt die Temperatur 50 bis 110°C, besonders bevorzugt 55 bis 90°C.

Die Reaktionszeiten können z.B. zwischen 30 Minuten und 15 Stunden betragen.

In einer bevorzugten Verfahrensweise wird das Kaliumfluorid und das Lösungsmittel(gemisch) vorgelegt. Dann wird im Verlauf von 5 bis 200 Minuten ein Sulfonsäureester der Formel (II) mit R¹ und R² = gegebenenfalls fluoriertem C₁-C₂-Alkyl zugetropft und der gebildete 2-Fluor-isobuttersäurealkylester unter vermindertem Druck kontinuierlich aus dem Reaktionsgemisch abdestilliert.

Wenn der nach Durchführung des erfindungsgemäßen Verfahrens isolierte 2-Fluor-isobuttersäurealkylester störenden Methacrylsäureester enthält, kann man diesen gegebenenfalls durch Chlor- oder Bromaddition abfangen. Man kann dabei beispielsweise so verfahren, daß man mit Brom titriert bis der Methacrylsäureester abreagiert hat, dann das Gemisch durch Waschen mit wenig Natriumbisulfit-Lösung entfärbt und in einer abschließenden Vakuumdestillation sehr reinen 2-Fluor-isobuttersäurealkylester erhält.

Das erfindungsgemäße Verfahren gestattet es unter milden Bedingungen mit gut zugänglichen Ausgangsprodukten und Reagenzien 2-Fluor-isobuttersäurealkylester in guten Ausbeuten herzustellen. Die eingangs bei den Verfahren des Standes der Technik geschilderten Nachteile treten nicht oder nur ganz untergeordnet auf. Insbesondere werden schlecht zugängliche, giftige und teure Ausgangsmaterialien und Reagenzien sowie die Notwendigkeit von besonders korrosionsresistenten Materialien für die Reaktion und die Aufarbeitung des Reaktionsgemisches vermieden.

Es ist ausgesprochen überraschend, daß sich die erfindungsgemäße Fluorierungsreaktion auch am tertiären C-Atom von Sulfonsäureestern der Formel (II) in guten Ausbeuten durchführen läßt, da hierbei ein S_{N}2-Mechanismus nicht möglich ist. Die erfindungsgemäße Umsetzung muß über einen weitgehend monomolekularen Carbeniumionen-artigen Übergangszustand ablaufen. Trotzdem ist die Eliminierung zu Methacrylsäureestern wider erwarten nur eine Nebenreaktion.

### Beispiele

### Beispiel 1

### a) 2-Methansulfonyloxy-2-methylpropionsäuremethylester

Zu einer unter Feuchtigkeitsauschluß bereiteten Lösung von 473 g 2-Methylmilchsäuremethylester und 607 g Triethylamin in 1 200 ml tert.-Butylmethylether (MTBE) wurde unter Kühlung bei max. 25°C eine Lösung von 550 g Methansulfonsäurechlorid in 400 ml MTBE getropft. Anschließend wurde langsam auf 60°C aufgeheizt und 1 Stunde bei dieser Temperatur nachgerührt. Dann wurde das Reaktionsgemisch auf 1,6 kg Eiswasser gegossen, die sich bildenden Phasen getrennt und die Wasserphase dreimal mit insgesamt 1 200 ml MTBE extrahiert. Die vereinigten organischen Phasen wurden zweimal mit insgesamt 1 600 ml 10-gew.-%iger Salzsäure und je einmal wäßriger Natriumhydrogencarbonat- und Natriumchlorid-Lösung gewaschen. Nach dem Trocknen über Magnesiumsulfat wurde das Gemisch bei max. 30°C am Rotationsverdampfer eingeengt und anschließend unter Rühren im Hochvakuum nachgetrocknet. Die Ausbeute betrug 616 g (= 80,1 % d. Theorie). Das Produkt wies eine Reinheit von 94 % (GC) auf.

### b) 2-Fluor-isobuttersäuremethylester

In einem 500 ml-Kolben mit mechanischen Rührer, Innenthermometer, Tropftrichter und Destillierbrücke wurden 93 g wasserfreies Kaliumfluorid bei 60°C in 220 ml trockenem Formamid gelöst. Der Druck wurde auf 15 mbar erniedrigt und dann im Verlaufe von 3 Stunden 78,5 g des gemäß a) hergestellten Sulfonsäureesters zugetropft. Das gebildete Produkt destillierte über. Nach beendeter Zugabe des Sulfonsäureesters wurde noch 3 Stunden nachreagieren gelassen. Die Ausbeute betrug 31,4 g Destillat, das lt. GC 75 Gew.-% 2-Fluor-isobuttersäuremethylester, 17 Gew.-% Methacrylsäuremethylester, 0,5 Gew.-% Formamid und Leichtsieder unbekannter Zusammensetzung enthielt. Die relative Ausbeute an gewünschtem Produkt betrug 50 % der Theorie.

### Beispiel 2

Es wurden analog Beispiel 1b) gearbeitet und 186 g Kaliumfluorid in 440 ml Formamid vorgelegt und mit 157 g des Sulfonsäureesters umgesetzt. Es wurden 67,7 g Rohprodukt der gleichen Zusammensetzung wie Beispiel 1b) erhalten. Dem Rohprodukt wurden dann unter Eiskühlung 18 g Brom zugetropft und anschließend durch Waschen mit 15 ml wäßriger Natriumbisulfitlösung entfärbt. Abschließend wurde bei 100 mbar über eine 10 cm-Vigreuxkolonne destilliert. Nach 4,3 g Vorlauf (77 gew.-%ig an gewünschtem Produkt) gingen bei 51 bis 53°C 44,6 g (= 46 % d. Theorie) 99 gew.-%iger 2-Fluor-isobuttersäuremethylester über. Als Nachlauf wurden noch 2,7 g 89 gew.-%iges Produkt erhalten. Die Gesamtausbeute an der gewünschten Verbindungen betrug also 52 % d. Theorie.

### Beispiel 3

Es wurde verfahren wie in Beispiel 2, jedoch wurde der Sulfonsäureester bei 90°C im Verlaufe von 1 Stunde zugetropft. So wurden 56,7 g Rohprodukt der Zusammensetzung 74 Gew.-% 2-Fluor-isobuttersäuremethylester, 21 Gew.-% Methacrylsäuremethylester und 2,7 Gew.-% Formamid erhalten. Die relative Ausbeute an der gewünschten Verbindung betrug 44 % d. Theorie.

## Patentansprüche

1. Verfahren zur Herstellung von 2-Fluor-isobuttersäurealkylestern der Formel (I) 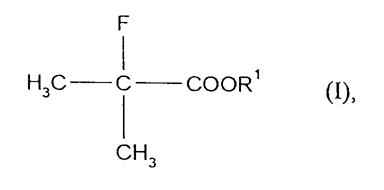 in der
R¹ einen gegebenenfalls verzweigten und/oder gegebenenfalls fluorierten Alkylrest mit 1 bis 4 Kohlenstoffatomen bedeutet,
dadurch gekennzeichnet ist, daß man einen Sulfonsäureester der Formel (II) 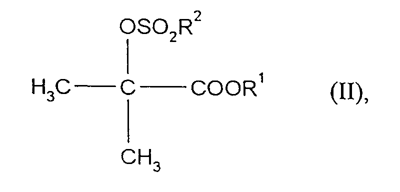 in der
R¹ die bei Formel (I) angegebene Bedeutung und
R² unabhängig von R¹ den gleichen Bedeutungsumfang wie R¹ hat,
**mit Kaliumfluorid in Gegenwart eines Lösungsmittels der Formel (III)**
R³-CO-NHR⁴ (III),
in der
R³ und R⁴ jeweils unabhängig voneinander Wasserstoff, Phenyl oder Alkyl mit 1 bis 4 Kohlenstoffatomen bedeuten,
umsetzt.

2. Verfahren nach Anspruch 1, dadurch gekennzeichnet, daß R¹ und R² unabhängig voneinander für Methyl, Trifluormethyl, Ethyl, n-Propyl, i-Propyl, n-Butyl, i-Butyl oder tert.-Butyl und R³ und R⁴ unabhängig voneinander für Wasserstoff oder Methyl stehen.

3. Verfahren nach Ansprüchen 1 und 2, dadurch gekennzeichnet, daß man als Sulfonsäureester der Formel (II) 2-Methansulfonyloxy-isobuttersäuremethylester einsetzt und daraus 2-Fluor-isobuttersäuremethylester herstellt.

4. Verfahren nach Ansprüchen 1 bis 3, dadurch gekennzeichnet, daß man Lösungsmittel Formamid einsetzt.

5. Verfahren nach Ansprüchen 1 bis 4, dadurch gekennzeichnet, daß man bezogen auf 1 Mol Sulfonsäureester der Formel (II) 1,05 bis 20 Mol Kaliumfluorid und die 2- bis 5fache Gewichtsmenge Lösungsmittel einsetzt.

6. Verfahren nach Ansprüchen 1 bis 5, dadurch gekennzeichnet, daß die Reaktionstemperatur im Bereich 40 bis 140°C liegt.

7. Verfahren nach Ansprüchen 1 bis 6, dadurch gekennzeichnet, daß man den gebildeten 2-Fluor-isobuttersäurealkylester durch Destillation aus dem Reaktionsgemisch entfernt.

## Claims

1. Process for the preparation of alkyl 2-fluoro-isobutyrates of the formula (I) 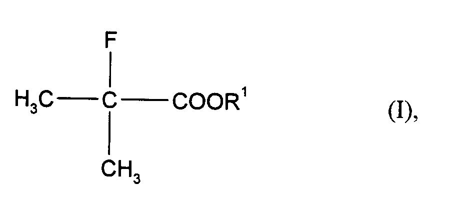 in which
R¹ denotes an unbranched or branched and/or unfluorinated or fluorinated alkyl radical having 1 to 4 carbon atoms,
characterized in that a sulphonic ester of the formula (II) 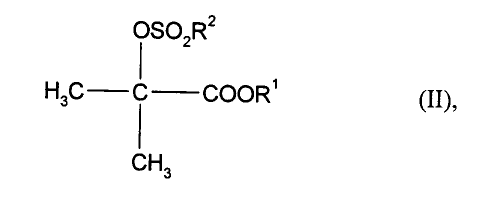 in which
R¹ has the meaning given under formula (I) and
R² independently of R¹, has the same scope of meanings as R¹,
is reacted with potassium fluoride in the presence of a solvent of the formula (III)
R³-CO-NHR⁴ (III),
in which
R³ and R⁴ , independently of each other, each denote hydrogen, phenyl or alkyl having 1 to 4 carbon atoms.

2. Process according to Claim 1, characterized in that R¹ and R², independently of each other, represent methyl, trifluoromethyl, ethyl, n-propyl, i-propyl, n-butyl, i-butyl or tert-butyl and R³ and R⁴, independently of each other, represent hydrogen or methyl.

3. Process according to Claims 1 and 2, characterized in that the sulphonic ester of the formula (II) used is methyl 2-methanesulphonyloxy-isobutyrate and methyl 2-fluoro-isobutyrate is prepared therefrom.

4. Process according to Claims 1 to 3, characterized in that the solvent used is formamide.

5. Process according to Claims 1 to 4, characterized in that, based on 1 mol of sulphonic ester of the formula (II), 1.05 to 20 mol of potassium fluoride and 2 to 5 times the amount by weight of solvent are used.

6. Process according to Claims 1 to 5, characterized in that the reaction temperature is in the range 40 to 140°C.

7. Process according to Claims 1 to 6, characterized in that the alkyl 2-fluoro-isobutyrate formed is removed from the reaction mixture by distillation.

## Revendications

1. Procédé de préparation d'esters alcoyliques d'acide 2-fluoroisobutyrique de formule (I) : 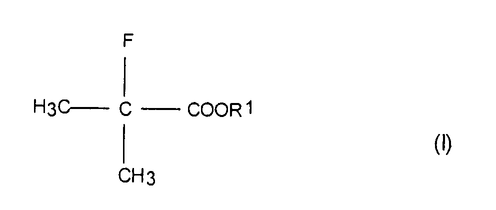 dans laquelle :
R¹ représente un reste alcoyle facultativement ramifié et/ou facultativement fluoré, ayant 1 à 4 atomes de carbone,
qui est caractérisé en ce que l'on fait réagir un ester d'acide sulfonique de formule (II) : 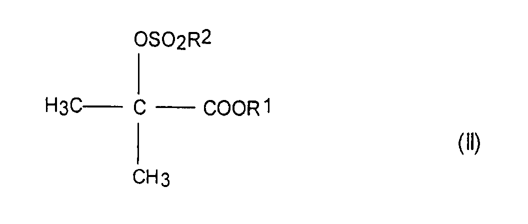 dans laquelle :
R¹ a la signification donnée pour la formule (I), et
R² a, indépendamment de R¹, la même étendue de signification que R¹,
avec du fluorure de potassium dans un solvant de formule (III) :
R³-CO-NHR⁴ (III)
dans laquelle :
R³ et R⁴ signifient, chacun indépendamment l'un de l'autre, hydrogène, phényle ou alcoyle ayant 1 à 4 atomes de carbone.

2. Procédé suivant la revendication 1, caractérisé en ce que R¹ et R² sont indépendamment l'un de l'autre, méthyle, trifluorométhyle, éthyle, n-propyle, i-propyle, n-butyle, i-butyle ou t-butyle et R³ et R⁴ sont indépendamment l'un de l'autre, hydrogène ou méthyle.

3. Procédé suivant les revendications 1 et 2, caractérisé en ce que l'on met en oeuyre comme ester d'acide sulfonique de formule (II), l'ester méthylique de l'acide 2-méthanesulfonyloxyisobutyrique et que l'on prépare ainsi, l'ester méthylique de l'acide 2-fluoroisobutyrique.

4. Procédé suivant les revendications 1 à 3, caractérisé en ce que l'on met en oeuvre le solvant formamide.

5. Procédé suivant les revendications 1 à 4, caractérisé en ce que l'on met en oeuvre, sur base de 1 mole d'ester d'acide sulfonique de formule (II), 1,05 à 20 moles de fluorure de potassium et 2 à 5 fois la quantité pondérale en solvant.

6. Procédé suivant les revendications 1 à 5, caractérisé en ce que la température de réaction se situe dans l'intervalle allant de 40 à 140° C.

7. Procédé suivant les revendications 1 à 6, caractérisé en ce que l'on sépare par distillation l'ester alcoylique de l'acide 2-fluoroisobutyrique formé du mélange réactionnel.
